# FASCICULE DE BREVET EUROPEEN

(11) **EP 1 023 894 B1**
(45) Date de publication et mention de la délivrance du brevet: **27.04.2005**
(21) Numéro de dépôt: 99403288.6
(22) Date de dépôt: 27.12.1999
(51) Int. Cl.: A61K 7/48, A61K 7/06, A61K 7/135

(54) **Blanchiment cosmétique avec un dérivé hydroxyphényloxime**
Kosmetische Aufhellung mit einem Hydroxyphenyloxim-Derivat
Cosmetic whitening with a hydroxyphenyloxime derivative

(30) Priorité: 27.01.1999 FR 9900882
(43) Date de publication de la demande: 02.08.2000
(73) Titulaire: L'OREAL, 75008 Paris (FR)
(72) Inventeur: Tuloup, Rémy, 75014 Paris (FR); Philippe, Michel, 91320 Wissous (FR)
(74) Mandataire: Kromer, Christophe

(56) Documents cités:
- WO-A-95/01157
- DE-A- 4 116 123
- FR-A- 2 765 801
- GB-A- 2 237 739
- NL-A- 8 900 621
- US-A- 4 762 705
- US-A- 4 816 487

## Description

La présente invention se rapporte à l'utilisation, dans une composition cosmétique topique, ou pour la fabrication d'une composition dermatologique topique, destinée à dépigmenter et/ou blanchir la peau humaine, les poils ou les cheveux, d'au moins un composé comportant un fragment phényloxime.

La couleur de la peau humaine est fonction de différents facteurs et notamment des saisons de l'année, de la race et du sexe, et elle est principalement déterminée par la nature et la concentration de mélanine produite par les mélanocytes. Les mélanocytes sont les cellules spécialisées qui par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

De la même manière, la couleur des poils et des cheveux est due à la mélanine, lorsque les poils ou les cheveux sont foncés, certaines personnes désirent voir ceux-ci plus clairs. Ceci est particulièrement intéressant pour les poils qui sont moins visibles lorsqu'ils sont clairs que lorsqu'ils sont foncés.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel, a été envisagé par de nombreux auteurs.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc et les leucodermies), à défaut de pouvoir repigmenter la peau lésée, on achève de dépigmenter les zones de peau normale résiduelle pour donner à l'ensemble de la peau une teinte blanche homogène.

Aussi, il subsiste le besoin d'un nouvel agent blanchissant de la peau humaine, des poils et/ou des cheveux à action aussi efficace que ceux connus, mais n'ayant pas leurs inconvénients, c'est-à-dire qui soit non irritant, non toxique et/ou non allergisant pour la peau et stable dans une composition.

La demanderesse a trouvé de manière inattendue que des composés comportant un fragment phényloxime présentent une activité dépigmentante, même à faibles concentrations, sans faire preuve de cytotoxicité.

Certains de ces composés ont déjà été décrits comme composés photoprotecteurs, destinés à protéger la peau contre le photoviellissement et l'érythème solaire (WO 95/01157), comme inhibiteurs de la tyrosinase dans des compositions orales pour le traitement du cancer, en association avec l'interféron (US-4,762,705) ou comme inhibiteurs de lipoxygénase, dans des compositions anti-inflammatoires (US-4,816,487). Toutefois, il n'a jamais été proposé, à la connaissance de la Demanderesse, de les utiliser en tant qu'agents dépigmentants ou dans des compositions dépigmentantes.

En outre, la salicylaldoxime a déjà été décrite comme dépigmentant (US-4,762,705), mais se différencie des composés objet de la présente invention par la présence d'un groupe hydroxy en position *ortho,* et non *para,* par rapport à la fonction imine.

La présente invention a donc pour objet l'utilisation pour blanchir la peau humaine, les poils ou les cheveux, dans une composition cosmétique comprenant un milieu cosmétiquement acceptable, destinée à une application topique sur la peau et/ou ses phanères, d'au moins un composé de formule (I) suivante : 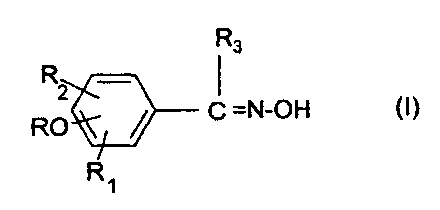 dans laquelle :
- R représente un groupement choisi parmi :
   - l'atome d'hydrogène ;
   - un groupement alkyle en C₁-C₂₄ linéaire ramifié ou cyclique, saturé ou insaturé, éventuellement hydroxylé par une ou plusieurs fonctions hydroxyles ;
   - un groupement aryle, substitué ou non par une ou plusieurs fonctions choisies parmi : -OH ; NH₂ ; -COOH ; -NO₂ ; -OR₅ avec R₅ = alkyle en C₁-C₂₄ ; -COOR₆ avec R₆ = alkyle en C₁-C₂₄ ; -NR₇R₈ avec R₇ = H ou alkyle en C₁-C₂₄, R₈ = H ou alkyle en C₁-C₂₄ ;
   - un groupement -COR₉, R₉ représentant un groupement alkyle en C₁-C₂₄ linéaire, ramifié ou cyclique, saturé ou insaturé, éventuellement hydroxylé par une ou plusieurs fonctions hydroxyles, ou un groupement aryle substitué ou non par une ou plusieurs fonctions choisies parmi -OH, -NH₂, -COOH, -NO₂, -OR₅, -COOR₆ et -NR₇R₈ dans lesquelles R₅, R₆, R₇ et R₈ ont la même définition que ci-dessus ;
- OR est en position *para* par rapport à la fonction imine ;
- R₁ et R₂, identiques ou différents, représentent un groupement choisi parmi :
   - l'atome d'hydrogène ;
   - un groupement alkyle en C₁-C₂₄, linéaire, ramifié ou cyclique, saturé ou insaturé, éventuellement hydroxylé par une ou plusieurs fonctions hydroxyles ;
   - un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus;
   - un groupement choisi parmi : -OH ; -OQ₁ ; -COQ₂ ; -COOQ₃ ; -NQ₄Q₅ ; -CONQ₆Q₇ ; -SQ₈ ; -CH₂OQ₉ ; Q₁, Q₂, Q₃, Q₄, Q₅, Q₆, Q₇, Q₈ et Q₉, identiques ou différents, étant choisis parmi l'atome d'hydrogène, les groupements alkyles en C₁-C₂₄, linéaires, ramifiés ou cycliques, saturés ou insaturés, éventuellement substitués par un ou plusieurs groupements hydroxyles, les aryles substitués ou non par une ou plusieurs fonctions choisies parmi : -OH, -NH₂, -COOH, -NO₂, -OR₅, -COOR₆, -NR₇R₈ dans lesquelles R₅, R₆, R₇ et R₈ ont la même définition que ci-dessus ; et
   - les résidus d'acides aminés et de carbohydrates cycliques ou non cycliques ;
- R₃ représente un groupement choisi parmi :
   - l'atome d'hydrogène ;
   - un groupement alkyle en C₁-C₂₄, linéaire, ramifié ou cyclique, saturé ou insaturé, éventuellement hydroxylé par une ou plusieurs fonctions hydroxyles ; et
   - un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus.

La présente invention a également pour objet l'utilisation d'au moins un composé de formule (I) ci-dessus pour la fabrication d'une composition dermatologique destinée à dépigmenter et/ou blanchir la peau humaine et/ou enlever les taches pigmentaires de la peau et/ou dépigmenter les poils et/ou les cheveux, comprenant un milieu dermatologiquement acceptable et destinée à une application topique sur la peau et/ou ses phanères.

Ces composés présentent l'avantage d'être faciles à obtenir. Ils peuvent être notamment obtenus en faisant réagir de l'hydroxylamine sur un aldéhyde (aldoximes) ou une cétone (cétoximes). Le réactif carbonylé peut éventuellement être utilisé sous forme d'acétal.

Parmi les radicaux alkyles linéaires saturés ayant de 1 à 24 atomes de carbone, on peut citer notamment les radicaux méthyle, éthyle, propyle, butyle, hexyle, octyle, nonyle, dodécyle, hexadécyle, béhényle et octadécyle.

Parmi les radicaux alkyles ramifiés saturés ayant de 1 à 24 atomes de carbone, on peut citer notamment les radicaux isopropyle, tertiobutyle, 2-éthyl-hexyle, 2-butyl-octyle, 2-hexyl-décyle.

Parmi les radicaux alkyles insaturés, on peut citer plus particulièrement le radical allyle.

Lorsque le radical alkyle est cyclique, on peut notamment citer le radical cyclohexyle, cholestéryle ou terbutylcyclohexyle.

Selon une forme de réalisation préférée, les composés de formule (I) de la présente invention sont ceux pour lesquels l'une au moins des conditions ci-dessous est respectée :
- R = H ou un groupement alkyle ayant de 1 à 6 atomes de carbone, ,
- R₁ = R₂ = H ou OH ou un groupement alkyle ayant de 1 à 6 atomes de carbone,
- R₃ = H ou un groupement alkyle ayant de 1 à 6 atomes de carbone.

Selon une forme de réalisation particulièrement préférée, les composés de formule (I) de la présente invention sont ceux pour lesquels l'une au moins des conditions ci-dessous est respectée :
- R = H,
- R₁ = R₂ = H,
- R₃ est un groupement alkyle ayant de 1 à 6 atomes de carbone, de préférence un groupe méthyle.

Le composé de formule (I) préféré est la para-hydroxyacétophénone oxime.

Par rapport aux composés de l'art antérieur connus comme dépigmentants, les composés de formule (I) présentent l'avantage d'être plus efficaces, comme il sera montré dans les tests ci-dessous.

La composition selon l'invention est appropriée à une utilisation topique et contient donc un milieu cosmétiquement ou dermatologiquement acceptable, c'est-à-dire compatible avec la peau, les poils ou les cheveux.

La quantité de dérivé de formule (I) dans la composition selon l'invention est bien entendu fonction de l'effet recherché et pourra donc varier dans une large mesure. Avantageusement, le ou les dérivés de formule (I) seront présents dans la composition en une quantité représentant de 0,001 à 10 %, et de préférence de 0,005 à 5 % du poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur les cheveux sous forme d'aérosol. Elle peut également se présenter sous forme solide, et par exemple sous forme de stick. Elle peut être utilisée comme produit de soin et/ou comme produit de maquillage. Elle peut également être sous une forme de shampooing ou d'après-shampooing.

De façon connue, la composition de l'invention peut contenir également les adjuvants habituels dans les domaines cosmétique et dermatologique, tels que des gélifiants hydrophiles ou lipophiles, des actifs hydrophiles ou lipophiles, des conservateurs, des antioxydants, des solvants, des parfums, des charges, des filtres, des pigments, des absorbeurs d'odeur et des matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Bien entendu, l'homme du métier veillera à choisir ces éventuels adjuvants, actifs ou non actifs, et/ou leur quantité, de telle manière que les propriétés avantageuses des composés selon l'invention ne soient pas, ou sensiblement pas, altérées par l'adjonction envisagée.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les polyols, les vitamines, les agents kératolytiques et/ou desquamants, les agents anti-inflammatoires, les agents apaisants et leurs mélanges. On peut également associer les composés de formule (I) à d'autres agents dépigmentants, tels que l'acide kojique ou l'hydroquinone et ses dérivés, ce qui permet d'utiliser ces derniers à des doses moins élevées. En cas d'incompatibilité, ces actifs et/ou les composés de formule (I) peuvent être incorporés dans des sphérules, notamment des vésicules ioniques ou non-ioniques et/ou des nanoparticules (nanocapsules et/ou nanosphères), de manière à les isoler les uns des autres dans la composition.

On peut aussi utiliser dans ces compositions des filtres UV à propriété lipophile ou hydrophile, tels que l'acide benzène 1,4-di(3-méthylidène-10-camphosulfonique), l'α-cyano-β,-β-diphénylacrylate de 2-éthylhexyle ou octocrylène, le butyl méthoxydibenzoylméthane, l'octyl méthoxycinnamate et/ou les oxydes de titane et de zinc.

L'invention va maintenant être illustrée à l'aide des exemples qui suivent. Les concentrations sont données en pourcentage en poids.

### Exemple de composé : Préparation de la para-hydroxyacétophénone oxime

On ajoute 1,1 équivalent de chlorhydrate d'hydroxylamine à une solution de *para*-hydroxyacétophénone dans la pyridine (dilution 16%). Le mélange réactionnel est porté à reflux jusqu'à disparition de l'acétophénone de départ, puis est versé dans de l'eau glacée. Le milieu est épuisé à l'acétate d'éthyle. Après séchage, la phase organique est éliminée et on obtient quantitativement l'oxime correspondante. L'analyse élémentaire est conforme à la structure.

### Test:

Un test biologique a mis en évidence l'activité dépigmentante des composés de formule (I).

Ce test correspond à celui décrit dans le brevet FR 2734825 déposé par la Demanderesse, ainsi que dans l'article de R. Schmidt, P. Krien et M. Régnier, Anal. Biochem., 235(2), 113-18, (1996). Ce test est ainsi réalisé sur coculture de kératinocytes et de mélanocytes.

Pour chaque composé testé, il est déterminé la valeur de IC50 qui correspond à la concentration micromolaire (µM) pour laquelle est observée 50% d'inhibition de la mélanogénèse.

Par ailleurs, une classe est donnée à chacun de ces composés pour leur activité dépigmentante maximale :
classe 1 : 10 à 30% d'inhibition de la mélanogénèse par rapport au témoin (même expérience sans composé à tester) ;
classe 2 : 30 à 60% d'inhibition de la mélanogénèse par rapport au témoin (même expérience sans composé à tester) ;
classe 3 : 60 à 100% d'inhibition de la mélanogénèse par rapport au témoin (même expérience sans composé à tester).

Les résultats sont rassemblés dans le tableau suivant :

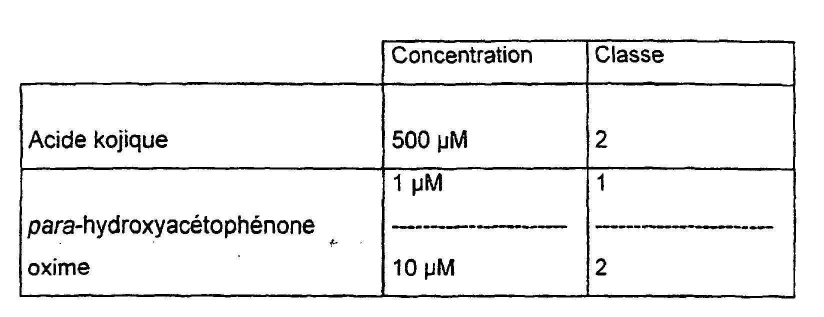

En outre, la valeur de IC50 est de 500 µM pour l'acide kojique et de 200 µM pour la *para*-hydroxyacétophénone oxime.

Ces composés de formule (I) présentent donc une plus grande efficacité dépigmentante que l'acide kojique. En outre, ils ont l'avantage de ne pas présenter de cytotoxicité à l'égard des kératinocytes et des mélanocytes, défaut majeur des dépigmentants déjà connus.

### Exemples de compositions

### Exemple 1 : Emulsion H/E

- Glycéryl stéarate et stéarate de PEG-100 3 %
- Alcool béhénylique 2,5 %
- Acide stéarique 1,5 %
- Cire d'abeille 4 %
- Triglycérides caprylique/caprique 7 %
- Polyisobutène hydrogéné 12 %
- Polyacrylamide/C₁₃₋₁₄ isoparaffin/laureth-7 (Sepigel 305) 0,5. %
- Glycérine 5 %
- Filtres UV 7 %
- Conservateurs 0,5 %
- Para-hydroxyacétophénone oxime 0,5 %
- Eau déminéralisée qsp 100 %

L'émulsion obtenue, utilisée en application quotidienne, permet d'obtenir un blanchiment de la peau.

### Exemple 2 : Fluide traitant

- Diglycol/CHDM/isophtalates/SIP copolymer 2 %
- Glycérine 5 %
- Huile d'abricot 14 %
- Cycloperitasiloxane 6 %
- Conservateurs 1 %
- Filtres UV 7 %
- Para-hydroxyacétophénone oxime 1 %
- Eau déminéralisée qsp 100 %

Le fluide obtenu peut être utilisé quotidiennement et est apte à dépigmenter la peau.

### Exemple 3 : Gel traitant

- Glycérine 5 %
- Copolymère acrylate / C10-30 alkylacrylate (Pemulen TR2) 0,5 %
- Conservateurs 0,1 %
- Ethanol 5 %
- Filtres UV 7 %
- Para-hydroxyacétophénone oxime 1 %
- Eau qsp 100 %

Le gel obtenu, utilisé sur les taches pigmentaires, permet de les atténuer voire de les faire disparaître.

## Revendications

1. Utilisation pour blanchir la peau humaine, les poils ou les cheveux, dans une composition cosmétique comprenant un milieu cosmétiquement acceptable, destinée à une application topique sur la peau et/ou ses phanères, d'au moins un dérivé de formule (I) suivante : 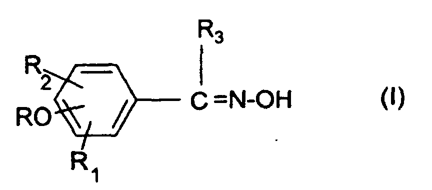 dans laquelle :
• R représente un groupement choisi parmi :
- l'atome d'hydrogène ;
- un groupement alkyle en C₁-C₂₄ linéaire ramifié ou cyclique, saturé ou insaturé, éventuellement hydroxylé par une ou plusieurs fonctions hydroxyles ;
- un groupement aryle, substitué ou non par une ou plusieurs fonctions choisies parmi : -OH ; NH₂ ; -COOH ; -NO₂ ; -OR₅ avec R₅ = alkyle en C₁-C₂₄ ; -COOR₆ avec R₆ = alkyle en C₁-C₂₄ ; -NR₇R₈ avec R₇ = H ou alkyle en C₁-C₂₄, R₈ = H ou alkyle en C₁-C₂₄ ;
- un groupement -COR₉, R₉ représentant un groupement alkyle en C₁-C₂₄ linéaire, ramifié ou cyclique, saturé ou insaturé, éventuellement hydroxylé par une ou plusieurs fonctions hydroxyles, ou un groupement aryle substitué ou non par une ou plusieurs fonctions choisies parmi -OH, -NH₂, -COOH, -NO₂, -OR₅, -COOR₆ ou -NR₇R₈ dans lesquelles R₅, R₆, R₇ et R₈ ont la même définition que ci-dessus ;
• OR est en position *para* par rapport à la fonction imine ;
• R₁ et R₂, identiques ou différents, représentent un groupement choisi parmi :
- l'atome d'hydrogène ;
- un groupement alkyle en C₁-C₂₄, linéaire, ramifié ou cyclique, saturé ou insaturé, éventuellement hydroxylé par une ou plusieurs fonctions hydroxyles ;
- un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus;
- un groupement choisi parmi : -OH ; -OQ₁ ; -COQ₂ ; -COOQ₃ ; -NQ₄Q₅ ; CONQ₆Q₇ ; -SQ₈ ; -CH₂OQ₉ ; Q₁, Q₂, Q₃, Q₄, Q₅, Q₆, Q₇, Q₈ et Q₉, identiques ou différents, étant choisis parmi l'atome d'hydrogène, les groupements alkyles en C₁-C₂₄, linéaires, ramifiés ou cycliques, saturés ou insaturés, éventuellement substitués par un ou plusieurs groupements hydroxyles, les aryles substitués ou non par une ou plusieurs fonctions choisies parmi : -OH, -NH₂, -COOH, -NO₂, -OR₅, -COOR₆, -NR₇R₈ dans lesquelles R₅, R₆, R₇ et R₈ ont la même définition que ci-dessus ; et
- les résidus d'acides aminés et de carbohydrates cycliques ou non cycliques ;
• R₃ représente un groupement choisi parmi :
- l'atome d'hydrogène ;
- un groupement alkyle en C₁-C₂₄, linéaire, ramifié ou cyclique, saturé ou insaturé, éventuellement hydroxylé par une ou plusieurs fonctions hydroxyles ; et
- un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus.

2. Utilisation, pour la fabrication d'une composition dermatologique destinée à dépigmenter la peau humaine et/ou enlever les taches pigmentaires de la peau et/ou dépigmenter les poils et/ou les cheveux, comprenant un milieu dermatologiquement acceptable, destinée à une application topique sur la peau et/ou ses phanères, d'au moins un dérivé de formule (I) suivante : 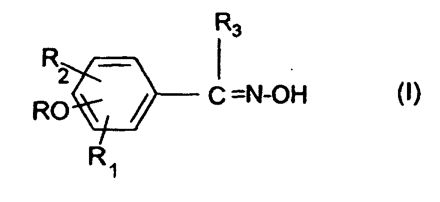 dans laquelle :
• R représente un groupement choisi parmi :
- l'atome d'hydrogène ;
- un groupement alkyle en C₁-C₂₄ linéaire ramifié ou cyclique, saturé ou insaturé, éventuellement hydroxylé par une ou plusieurs fonctions hydroxyles;
- un groupement aryle, substitué ou non par une ou plusieurs fonctions choisies parmi : -OH ; NH₂ ; -COOH ; -NO₂ ; -OR₅ avec R₅ = alkyle en C₁-C₂₄ ; -COOR₆ avec R₆ = alkyle en C₁-C₂₄ ; -NR₇R₈ avec R₇ = H ou alkyle en C₁-C₂₄, R₈ = H ou alkyle en C₁-C₂₄ ;
- un groupement -COR₉, R₉ représentant un groupement alkyle en C₁-C₂₄ linéaire, ramifié ou cyclique, saturé ou insaturé, éventuellement hydroxylé par une ou plusieurs fonctions hydroxyles, ou un groupement aryle substitué ou non par une ou plusieurs fonctions choisies parmi -OH, -NH₂, -COOH, -NO₂, -OR₅, -COOR₆ ou -NR₇R₈ dans lesquelles R₅, R₆, R₇ et R₈ ont la même définition que ci-dessus ;
• OR est en position para par rapport à la fonction imine ;
• R₁ et R₂, identiques ou différents, représentent un groupement choisi parmi :
- l'atome d'hydrogène ;
- un groupement alkyle en C₁-C₂₄, linéaire, ramifié ou cyclique, saturé ou insaturé, éventuellement hydroxylé par une ou plusieurs fonctions hydroxyles ;
- un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus;
- un groupement choisi parmi : -OH ; -OQ₁ ; -COQ₂ ; -COOQ₃ ; -NQ₄Q₅ ; CONQ₆Q₇ ; -SQ₈ ; -CH₂OQ₉ ; Q₁, Q₂, Q₃, Q₄, Q₅, Q₆, Q₇, Q₈ et Q₉, identiques ou différents, étant choisis parmi l'atome d'hydrogène, les groupements alkyles en C₁-C₂₄, linéaires, ramifiés ou cycliques, saturés ou insaturés, éventuellement substitués par un ou plusieurs groupements hydroxyles, les aryles substitués ou non par une ou plusieurs fonctions choisies parmi : -OH, -NH₂, -COOH, -NO₂, -OR₅, -COOR₆, -NR₇R₈ dans lesquelles R₅, R₆, R₇ et R₈ ont la même définition que ci-dessus ; et
- les résidus d'acides aminés et de carbohydrates cycliques ou non cycliques ;
• R₃ représente un groupement choisi parmi :
- l'atome d'hydrogène ;
- un groupement alkyle en C₁-C₂₄, linéaire, ramifié ou cyclique, saturé ou insaturé, éventuellement hydroxylé par une ou plusieurs fonctions hydroxyles ; et
- un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus.

3. Utilisation selon la revendication 1 ou 2, **caractérisée en ce que** ledit dérivé présente l'une au moins des conditions ci-dessous :
- R = H ou un groupement alkyle ayant de 1 à 6 atomes de carbone,
- R₁ = R₂ = H ou OH ou un groupement alkyle ayant de 1 à 6 atomes de carbone,
- R₃ = H ou un groupement alkyle ayant de 1 à 6 atomes de carbone.

4. Utilisation selon la revendication 3, **caractérisée en ce que** ledit dérivé présente au moins l'une des conditions ci-dessous :
- R = H,
- R₁ = R₂ = H,
- R₃ = CH₃.

5. Utilisation selon la revendication 4, **caractérisée en ce que** ledit dérivé est la *para*-hydroxyacétophénone oxime.

6. Utilisation selon l'une quelconque des revendications 1 à 5, **caractérisée en ce que** le dérivé de formule (I) est présent en une quantité allant de 0,001 à 10%, et de préférence de 0,005 à 5%, du poids total de la composition.

7. Utilisation selon l'une quelconque des revendications 1 à 6, **caractérisée en ce que** la composition comprend en outre au moins un actif choisi parmi les agents kératolytiques et/ou desquamants, les filtres UV et les autres agents dépigmentants.

## Patentansprüche

1. Verwendung mindestens eines Derivats der folgenden Formel (I) in einer kosmetischen Zusammensetzung, die ein kosmetisch akzeptables Medium enthält und für eine topische Anwendung auf die Haut und/oder die Hautanhangsgebilde vorgesehen ist, zum Bleichen der menschlichen Haut, der Körperhaare oder der Haare: 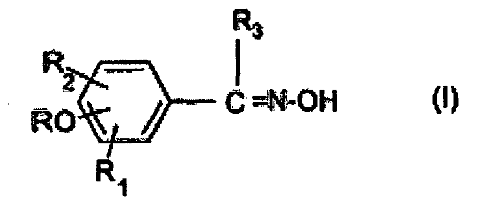 worin bedeuten:
• R eine Gruppe, die ausgewählt ist unter:
- einem Wasserstoffatom;
- einer geradkettigen, verzweigten oder cyclischen, gesättigten oder ungesättigten C₁₋₂₄-Alkylgruppe, die gegebenenfalls mit einer oder mehreren Hydroxyfunktionen hydroxyliert ist;
- einer Arylgruppe, die gegebenenfalls mit einer oder mehreren Gruppen substituiert ist, die ausgewählt sind unter: -OH; NH₂; -COOH; -NO₂; -OR₅ mit R₅ = C₁₋₂₄-Alkyl; -COOR₆ mit R₆ = C₁₋₂₄-Alkyl; -NR₇R₈ mit R₇ = H oder C₁₋₂₄-Alkyl; R₈ = H oder C₁₋₂₄-Alkyl;
- einer Gruppe -COR₉, wobei R₉ eine geradkettige, verzweigte oder cyclische, gesättigte oder ungesättigte C₁₋₂₄-Alkylgruppe bedeutet, die gegebenenfalls mit einer oder mehreren Hydroxyfunktionen substituiert ist, oder eine Arylgruppe, die unsubstituiert vorliegt oder mit einer oder mehreren Funktionen substituiert ist, die ausgewählt sind unter -OH, -NH₂, -COOH, -NO₂, -OR₅, -COOR₆ und -NR₇R₈, wobei R₅, R₆, R₇ und R₈ die oben angegebenen Bedeutungen aufweisen;
• OR befindet sich in para-Stellung zur Iminfunktion;
• R₁ und R₂, die gleich oder verschieden sind, bedeuten eine Gruppe, die ausgewählt ist unter:
- einem Wasserstoffatom;
- einer geradkettigen, verzweigten oder cyclischen, gesättigten oder ungesättigten C1-24-Alkylgruppe, die gegebenenfalls mit einer oder mehreren Hydroxygruppen hydroxyliert ist;
- einer Arylgruppe, die unsubstituiert vorliegt oder mit einer oder mehreren Funktionen substituiert ist, die ausgewählt sind unter -OH; NH₂; -COOH; -NO₂; -OR₅, -COOR₆, -NR₇R₈, wobei R₅, R₆, R₇ und R₈ die oben angegebenen Bedeutungen aufweisen;
- einer Gruppe, die ausgewählt ist unter: -OH; -OQ₁; -COQ₂; -COOQ₃; -NQ₄Q₅; CONQ₆Q₇; -SQ₈; -CH₂OQ₉; wobei Q₁, Q₂, Q₃, Q₄, Q₅, Q₆, Q₇, Q₈ und Q₉, die gleich oder verschieden sind, unter Wasserstoff, geradkettigen, verzweigten oder cyclischen, gesättigten oder ungesättigten C₁₋₂₄-Alkylgruppen, die gegebenenfalls mit einer oder mehreren Hydroxygruppen substituiert sind, und unsubstituierten oder mit einer oder mehreren der folgenden Gruppen substituierten Arylgruppen ausgewählt sind: -OH, -NH₂, -COOH, -NO₂, -OR₅, -COOR₆, -NR₇R₈, wobei Rs, R₆, R₇ und R₈ die oben angegebenen Bedeutungen aufweisen; und
- den Aminosäureresten und Kohlehydratresten, die cyclisch oder nicht cyclisch sind;
• R₃ eine Gruppe, die ausgewählt ist unter:
- einem Wasserstoffatom;
- einer geradkettigen, verzweigten oder cyclischen, gesättigten oder ungesättigten C₁₋₂₄-Alkylgruppe, die gegebenenfalls mit einer oder mehreren Hydroxygruppen hydroxyliert ist; und
- einer Arylgruppe, die unsubstituiert vorliegt oder mit einer oder mehreren der folgenden Funktionen substituiert ist: -OH; NH₂; -COOH; -NO₂; -OR₅, -COOR₆, -NR₇R₈, wobei R₅, R₆, R₇ und R₈ die oben angegebenen Bedeutungen aufweisen.

2. Verwendung mindestens eines Derivats der folgenden Formel (I) zur Herstellung einer dermatologischen Zusammensetzung, die dazu vorgesehen ist, die menschliche Haut zu depigmentieren und/oder Pigmentflecken der Haut zu entfernen und/oder die Körperhaare und/oder Haare zu depigmentieren, die ein dermatologisch akzeptables Medium enthält und die für eine topische Anwendung auf die Haut und/oder die Hautanhangsgebilde vorgesehen ist: 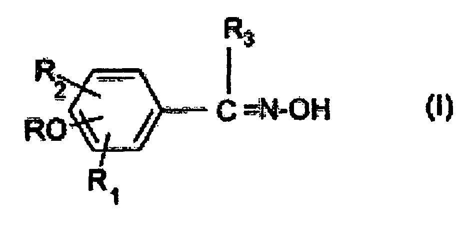 worin bedeuten:
• R eine Gruppe, die ausgewählt ist unter:
- einem Wasserstoffatom;
- einer geradkettigen, verzweigten oder cyclischen, gesättigten oder ungesättigten C₁₋₂₄-Alkylgruppe, die gegebenenfalls mit einer oder mehreren Hydroxyfunktionen hydroxyliert ist;
- einer Arylgruppe, die gegebenenfalls mit einer oder mehreren Gruppen substituiert ist, die ausgewählt sind unter: -OH; NH₂; -COOH; -NO₂; -OR₅ mit R₅ = C₁₋₂₄-Alkyl; -COOR₆ mit R₆ = C₁₋₂₄-Alkyl; -NR₇R₈ mit R₇ = H oder C₁₋₂₄-Alkyl; R₈ = H oder C₁₋₂₄-Alkyl;
- einer Gruppe -COR₉, wobei R₉ eine geradkettige, verzweigte oder cyclische, gesättigte oder ungesättigte C₁₋₂₄-Alkylgruppe bedeutet, die gegebenenfalls mit einer oder mehreren Hydroxyfunktionen substituiert ist, oder eine Arylgruppe, die unsubstituiert vorliegt oder mit einer oder mehreren Funktionen substituiert ist, die ausgewählt sind unter -OH, -NH₂, -COOH, -NO₂, -OR₅, -COOR₆ und -NR₇R₈, wobei R₅, R₆, R₇ und R₈ die oben angegebenen Bedeutungen aufweisen;
• OR befindet sich in para-Stellung zur Iminfunktion;
• R₁ und R₂, die gleich oder verschieden sind, bedeuten eine Gruppe, die ausgewählt ist unter:
- einem Wasserstoffatom;
- einer geradkettigen, verzweigten oder cyclischen, gesättigten oder ungesättigten C₁₋₂₄-Alkylgruppe, die gegebenenfalls mit einer oder mehreren Hydroxygruppen hydroxyliert ist;
- einer Arylgruppe, die unsubstituiert vorliegt oder mit einer oder mehreren Funktionen substituiert ist, die ausgewählt sind unter -OH; NH2; -COOH; -NO₂; -OR₅, -COOR₆, -NR₇R₈, wobei R₅, R₆, R₇ und R₈ die oben angegebenen Bedeutungen aufweisen;
- einer Gruppe, die ausgewählt ist unter: -OH; -OQ₁; -COQ₂; -COOQ₃; -NQ₄Q₅; CONQ₆Q₇; -SQ₈; -CH₂OQ₉; wobei Q₁, Q₂, Q₃, Q₄, Q₅, Q₆, Q₇, Q₈ und Q₉, die gleich oder verschieden sind, unter Wasserstoff, geradkettigen, verzweigten oder cyclischen, gesättigten oder ungesättigten C₁₋₂₄-Alkylgruppen, die gegebenenfalls mit einer oder mehreren Hydroxygruppen substituiert sind, und unsubstituierten oder mit einer oder mehreren der folgenden Gruppen substituierten Arylgruppen ausgewählt sind: -OH, -NH₂, -COOH, -NO₂, -OR₅, -COOR₆, -NR₇R₈, wobei R₅, R₆, R₇ und R₈ die oben angegebenen Bedeutungen aufweisen; und
- den Aminosäureresten und Kohlehydratresten, die cyclisch oder nicht cyclisch sind;
• R₃ eine Gruppe, die ausgewählt ist unter:
- einem Wasserstoffatom;
- einer geradkettigen, verzweigten oder cyclischen, gesättigten oder ungesättigten C₁₋₂₄-Alkylgruppe, die gegebenenfalls mit einer oder mehreren Hydroxygruppen hydroxyliert ist; und
- einer Arylgruppe, die unsubstituiert vorliegt oder mit einer oder mehreren der folgenden Funktionen substituiert ist: -OH; NH₂; -COOH; -NO₂; -OR₅, -COOR₆, -NR₇R₈, wobei R₅, R₆, R₇ und R₈ die oben angegebenen Bedeutungen aufweisen.

3. Verwendung nach Anspruch 1 oder 2, **dadurch gekennzeichnet, dass** das Derivat mindestens eine der folgenden Bedingungen erfüllt:
- R = H oder eine Alkylgruppe mit 1 bis 6 Kohlenstoffatomen,
- R₁ = R₂ = H oder OH oder eine Alkylgruppe mit 1 bis 6 Kohlenstoffatomen,
- R₃ = H oder eine Alkylgruppe mit 1 bis 6 Kohlenstoffatomen.

4. Verwendung nach Anspruch 3, **dadurch gekennzeichnet, dass** das Derivat mindestens eine der folgenden Bedingungen erfüllt:
- R = H,
- R₁ = R₂ = H,
- R₃ = CH₃.

5. Verwendung nach Anspruch 4, **dadurch gekennzeichnet, dass** es sich bei dem Derivat um das para-Hydroxyacetophenonoxim handelt.

6. Verwendung nach einem der Ansprüche 1 bis 5, **dadurch gekennzeichnet, dass** das Derivat der Formel (I) in einer Menge von 0,001 bis 10 % und vorzugsweise 0,005 bis 5 % des Gesamtgewichts der Zusammensetzung enthalten ist.

7. Verwendung nach einem der Ansprüche 1 bis 6, **dadurch gekennzeichnet, dass** die Zusammensetzung ferner mindestens einen Wirkstoff enthält, der unter den Keratolytika und/oder abschuppenden Wirkstoffen, UV-Filtern und weiteren depigmentierenden Wirkstoffen ausgewählt ist.

## Claims

1. Use, for bleaching human skin, body hair or head hair, in a cosmetic composition comprising a cosmetically acceptable medium, intended for topical application to the skin and/or its integuments, of at least one derivative of formula (I) below: 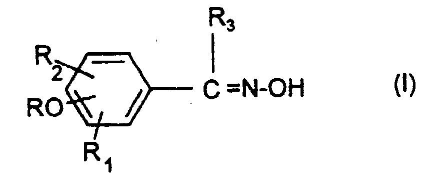 in which:
• R represents a group chosen from:
- a hydrogen atom;
- a linear, branched or cyclic, saturated or unsaturated C₁-C₂₄ alkyl group, optionally hydroxylated with one or more hydroxyl functions;
- an aryl group, which may be unsubstituted or substituted with one or more functions chosen from: -OH; NH₂; -COOH; -NO₂; -OR₅ with R₅ = C₁-C₂₄ alkyl; -COOR₆ with R₆ = C₁-C₂₄ alkyl; -NR₇R₈ with R₇ = H or C₁-C₂₄ alkyl, R₈ = H or C₁-C₂₄ alkyl;
- a group -COR₉, R₉ representing a linear, branched or cyclic, saturated or unsaturated C₁-C₂₄ alkyl group, optionally hydroxylated with one or more hydroxyl functions, or an aryl group which may be unsubstituted or substituted with one or more functions chosen from -OH, -NH₂, -COOH, -NO₂, -OR₅, -COOR₆ and -NR₇R₈ in which R₅, R₆, R₇ and R₈ have the same definition as above;
• OR is in the para position relative to the imine function;
• R₁ and R₂, which may be identical or different, represent a group chosen from:
- a hydrogen atom;
- a linear, branched or cyclic, saturated or unsaturated C₁-C₂₄ alkyl group, optionally hydroxylated with one or more hydroxyl functions;
- an aryl group, which may be unsubstituted or substituted with one or more functions chosen from -OH, -NH₂, -COOH, -NO₂, -OR₅, -COOR₆ and -NR₇R₈ in which R₅, R₆, R₇ and R₈ have the same definition as above;
- a group chosen from: -OH; -OQ₁; -COQ₂; -COOQ₃; -NQ₄Q₅; -CONQ₆Q₇; -SQ₈; -CH₂OQ₉; Q₁, Q₂, Q₃, Q₄, Q₅, Q₆, Q₇, Q₈ and Q₉, which may be identical or different, being chosen from a hydrogen atom, linear, branched or cyclic, saturated or unsaturated C₁-C₂₄ alkyl groups, optionally substituted with one or more hydroxyl groups, aryl groups which may be unsubstituted or substituted with one or more functions chosen from: -OH, -NH₂, -COOH, -NO₂, -OR₅, -COOR₆ and -NR₇R₈ in which R₅, R₆, R₇ and R₈ have the same definition as above; and
- amino acid residues and cyclic or non-cyclic carbohydrate residues;
• R₃ represents a group chosen from:
- a hydrogen atom;
- a linear, branched or cyclic, saturated or unsaturated C₁-C₂₄ alkyl group, optionally hydroxylated with one or more hydroxyl functions; and
- an aryl group, which may be unsubstituted or substituted with one or more functions chosen from -OH, -NH₂, -COOH, -NO₂, -OR₅, -COOR₆ and -NR₇R₈ in which R₅, R₆, R₇ and R₈ have the same definition as above.

2. Use, for the manufacture of a dermatological composition intended for depigmenting human skin and/or for removing pigmentation marks from the skin and/or for depigmenting body hair and/or head hair, comprising a dermatologically acceptable medium, which is intended for topical application to the skin and/or its integuments, of at least one derivative of formula (I) below: 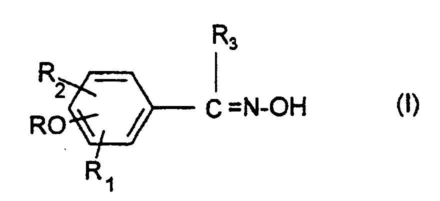 in which:
• R represents a group chosen from:
- a hydrogen atom;
- a linear, branched or cyclic, saturated or unsaturated C₁-C₂₄ alkyl group, optionally hydroxylated with one or more hydroxyl functions;
- an aryl group, which may be unsubstituted or substituted with one or more functions chosen from: -OH; NH₂; -COOH; -NO₂; -OR₅ with R₅ = C₁-C₂₄ alkyl; -COOR₆ with R₆ = C₁-C₂₄ alkyl; -NR₇R₈ with R₇ = H or C₁-C₂₄ alkyl, R₈ = H or C₁-C₂₄ alkyl;
- a group -COR₉, R₉ representing a linear, branched or cyclic, saturated or unsaturated C₁-C₂₄ alkyl group, optionally hydroxylated with one or more hydroxyl functions, or an aryl group which may be unsubstituted or substituted with one or more functions chosen from -OH, -NH₂, -COOH, -NO₂, -OR₅, -COOR₆ and -NR₇R₈ in which R₅, R₆, R₇ and R₈ have the same definition as above;
• OR is in the para position relative to the imine function;
• R₁ and R₂, which may be identical or different, represent a group chosen from:
- a hydrogen atom;
- a linear, branched or cyclic, saturated or unsaturated C₁-C₂₄ alkyl group, optionally hydroxylated with one or more hydroxyl functions;
- an aryl group, which may be unsubstituted or substituted with one or more functions chosen from -OH, -NH₂, -COOH, -NO₂, -OR₅, -COOR₆ and -NR₇R₈ in which R₅, R₆, R₇ and R₈ have the same definition as above;
- a group chosen from: -OH; -OQ₁; -COQ₂; -COOQ₃; -NQ₄Q₅; -CONQ₆Q₇; -SQ₈; -CH₂OQ₉; Q₁, Q₂, Q₃, Q₄, Q₅, Q₆, Q₇, Q₈ and Q₉, which may be identical or different, being chosen from a hydrogen atom, linear, branched or cyclic, saturated or unsaturated C₁-C₂₄ alkyl groups, optionally substituted with one or more hydroxyl groups, aryl groups which may be unsubstituted or substituted with one or more functions chosen from: -OH, -NH₂, -COOH, -NO₂, -OR₅, -COOR₆ and -NR₇R₈ in which R₅, R₆, R₇ and R₈ have the same definition as above; and
- amino acid residues and cyclic or non-cyclic carbohydrate residues;
• R₃ represents a group chosen from:
- a hydrogen atom;
- a linear, branched or cyclic, saturated or unsaturated C₁-C₂₄ alkyl group, optionally hydroxylated with one or more hydroxyl functions; and
- an aryl group, which may be unsubstituted or substituted with one or more functions chosen from -OH, -NH₂, -COOH, -NO₂, -OR₅, -COOR₆ and -NR₇R₈ in which R₅, R₆, R₇ and R₈ have the same definition as above.

3. Use according to Claim 1 or 2, **characterized in that** the said derivative displays at least one of the conditions below:
- R = H or an alkyl group containing from 1 to 6 carbon atoms,
- R₁ = R₂ = H or OH or an alkyl group containing from 1 to 6 carbon atoms,
- R₃ = H or an alkyl group containing from 1 to 6 carbon atoms.

4. Use according to Claim 3, **characterized in that** the said derivative displays at least one of the conditions below:
- R = H,
- R₁ = R₂ = H,
- R₃ = CH₃.

5. Use according to Claim 4, **characterized in that** the said derivative is para-hydroxyacetophenone oxime.

6. Use according to any one of Claims 1 to 5, **characterized in that** the derivative of formula (I) is present in an amount ranging from 0.001% to 10% and preferably from 0.005% to 5% relative to the total weight of the composition.

7. Use according to any one of Claims 1 to 6, **characterized in that** the composition also comprises at least one active agent chosen from keratolytic agents and/or desquamating agents, UV-screening agents and other depigmenting agents.
